(19) Europäisches Patentamt
European Patent Office
Office européen des brevets

(11) **EP 4 219 438 B1**

(12) **EUROPEAN PATENT SPECIFICATION**

(45) Date of publication and mention
of the grant of the patent:
**21.05.2025 Bulletin 2025/21**

(21) Application number: **21900668.1**

(22) Date of filing: **02.12.2021**

(51) International Patent Classification (IPC):
*C07C 45/50* (2006.01)      *B01J 31/02* (2006.01)
*C07B 61/00* (2006.01)      *C07C 47/02* (2006.01)
*B01J 31/20* (2006.01)

(52) Cooperative Patent Classification (CPC):
(C-Sets available)
**C07C 45/50; B01J 31/20;** B01J 2231/321;
B01J 2523/845                          (Cont.)

(86) International application number:
**PCT/JP2021/044289**

(87) International publication number:
**WO 2022/118917 (09.06.2022 Gazette 2022/23)**

(54) **METHOD FOR PRODUCING ALDEHYDE**

VERFAHREN ZUR HERSTELLUNG VON ALDEHYD

PROCÉDÉ DE PRODUCTION D'ALDÉHYDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.12.2020 JP 2020201432**

(43) Date of publication of application:
**02.08.2023 Bulletin 2023/31**

(73) Proprietor: **KH Neochem Co., Ltd.
Tokyo 103-0022 (JP)**

(72) Inventors:
• **KISHIMOTO, Shigehisa**
  **Ichihara-City, Chiba 2908560 (JP)**
• **OHGATA, Yusuke**
  **Ichihara-City, Chiba 2908560 (JP)**
• **KANEDA, Youichi**
  **Ichihara-City, Chiba 2908560 (JP)**
• **KITABATAKE, Kouji**
  **Ichihara-City, Chiba 2908560 (JP)**

(74) Representative: **V.O.
P.O. Box 87930
2508 DH Den Haag (NL)**

(56) References cited:
WO-A1-2013/014959      WO-A1-2014/005854
CN-A- 111 470 962      CN-A- 111 909 014
GB-A- 702 204          GB-A- 702 204
JP-A- 2000 344 695     JP-A- 2001 187 759
JP-A- 2001 509 496     JP-A- 2002 053 501
US-A1- 2002 026 087    US-B1- 6 365 783

(52) Cooperative Patent Classification (CPC): (Cont.)

C-Sets
**C07C 45/50, C07C 47/02**

**Description**

Technical Field

The present invention relates to a method for producing 3,5,5-trimethylhexanal that is useful as a raw material or the like of carboxylic acid or the like that becomes a raw material of a refrigerating machine oil, etc.

Background Art

A method for producing a saturated aliphatic aldehyde, the number of carbon atoms of which is larger than that of a monoolefin by one, and a saturated aliphatic alcohol by allowing a monoolefin to react with carbon monoxide and hydrogen (oxo reaction) has been conventionally widely used as an oxo synthesis process. The aldehyde, the number of carbon atoms of which is larger than that of a monoolefin by one and which is obtained by oxo reaction using a monoolefin as a raw material, becomes a raw material of carboxylic acid that is useful as a raw material or the like of a refrigerating machine oil, etc. The carboxylic acid is obtained by oxidation of an aldehyde, and therefore, in order to obtain desired carboxylic acid, it is desired to obtain an aldehyde corresponding thereto selectively and in a high yield.

As the method for producing an aldehyde, a method in which a rhodium-based catalyst or a cobalt-based catalyst is used is known, but rhodium is expensive as compared with cobalt. Therefore, it is desired to obtain an aldehyde selectively and in a high yield using a cobalt-based catalyst. Known is a method in which, for example, a cobalt carbonyl-based catalyst among the cobalt-based catalysts is used. This method has advantages of a high reaction rate and easy recovery of a catalyst. In this method, however, there is a problem that a by-product is produced in a large amount, as described in Patent Literature 1. That is to say, in the initial stage of the reaction, a saturated aliphatic aldehyde, the number of carbon atoms of which is larger than that of a monoolefin by one, is obtained as a main product from a monoolefin, but this aldehyde is converted into a saturated aliphatic alcohol, the number of carbon atoms of which is larger than that of a monoolefin by one, through hydrogenation reaction as a second-order reaction, so that this causes decrease in yield and purity of an aldehyde. Moreover, the alcohol reacts with the aldehyde to produce a by-product derived from an acetal, and therefore, the aldehyde yield is further deceased. Accordingly, it has been desired to suppress such a second-order reaction and to obtain an aldehyde selectively and in a high yield, and on this account, it is a problem of the above method to suppress production of an alcohol.

As a method to solve the above problem, a method for suppressing production of an acetal or the like by allowing a monoolefin, carbon monoxide and hydrogen to react with one another in the presence of a cobalt carbonyl-based catalyst, subsequently separating unreacted olefin from a reaction product, and further allowing the unreacted olefin, carbon monoxide and hydrogen to react with one another in the presence of a cobalt catalyst and water is disclosed in Patent Literature 1, and specifically, a butene dimer or the like is used as a raw material.

In Patent Literature 2, a method for suppressing side reaction by allowing an olefin, carbon monoxide and hydrogen to react with one another in the presence of a cobalt catalyst, subsequently separating an olefin and low-boiling substances containing paraffin from a reaction product, and further allowing the unreacted olefin, carbon monoxide and hydrogen to react with one another in the presence of a cobalt catalyst is disclosed, and specifically, di-n-butene or the like is used as a raw material.

In Patent Literature 3, a method for producing an oxygen-containing compound, including allowing an olefin, carbon monoxide and hydrogen to react with one another in the presence of a cobalt catalyst, subsequently separating an unreacted monoolefin from a reaction product, and allowing the unreacted monoolefin, carbon monoxide and hydrogen to react with one another in the presence of a cobalt catalyst is disclosed, and specifically, a codimer of a butene isomer, or the like is used as a raw material.

Citation List

Patent Literature

Patent Literature 1
Japanese Patent No. 4368454
Patent Literature 2
Japanese Patent Laid-Open No. 2002-53501
Patent Literature 3
UK Patent No. 702204

Summary of Invention

Technical Problem

In the method described in Patent Literature 1, however, the amount of an alcohol produced is large, and the aldehyde selectivity is low, so that the method is practically unsatisfactory.

In the method described in Patent Literature 2 also, the amount of an alcohol produced is large, and the aldehyde selectivity is low, though production of a high-boiling component such as an acetal can be suppressed, so that the method is practically unsatisfactory.

In Patent Literature 3, a reaction using isobutylene as a raw material is also described, but it is not described to allow unreacted isobutylene to react with carbon monoxide and hydrogen again.

In the light of the above circumstances, an object of the present invention is to provide a method for producing, selectively and in a high yield, 3,5,5-trimethylhexanal that is useful as a raw material or the like of carboxylic acid or the like that becomes a raw material of a refrigerating machine oil, etc.

Solution to Problem

[1] A method for producing 3,5,5-trimethylhexanal by allowing diisobutylene (DIB), carbon monoxide and hydrogen to react with one another in the presence of a cobalt-based catalyst, comprising:

a step of allowing DIB to react with carbon monoxide and hydrogen in the presence of a cobalt-based catalyst until a conversion of DIB reaches 50 to 85% (first stage reaction step);

a step of separating unreacted DIB from a reaction product obtained in the first stage reaction step (unreacted DIB separation step); and

a step of allowing the unreacted DIB separated to react with carbon monoxide and hydrogen in the presence of a cobalt-based catalyst (second stage reaction step).

[2] The method for producing 3,5,5-trimethylhexanal according to the above [1], wherein in the second stage reaction step, a reaction temperature (°C) x and a cobalt concentration (g/kg) y based on DIB satisfy the following expressions (1) and (2):

$$(1)\quad y \leq 8.0 \times 10^6 e^{-0.09x}$$

$$(2)\quad 0.1 \leq y \leq 20.$$

[3] The method for producing 3,5,5-trimethylhexanal according to the above [2], wherein in the second stage reaction step, the cobalt concentration (g/kg) y based on DIB satisfies the following expression (3):

(3) $0.1 \leq y \leq 15$.

[4] The method for producing 3,5,5-trimethylhexanal according to any one of the above [1] to [3], wherein the reaction temperature in the second stage reaction step is 180°C or less.

[5] A method for producing 3,5,5-trimethylhexanoic acid, comprising a step of oxidizing 3,5,5-trimethylhexanal obtained in the production method according to any one of the above [1] to [4].

[6] A method for suppressing production of 3,5,5-trimethylhexanol, in a method for producing 3,5,5-trimethylhexanal by allowing diisobutylene (DIB), carbon monoxide and hydrogen to react with one another in the presence of a cobalt-based catalyst, comprising:

a step of allowing DIB to react with carbon monoxide and hydrogen in the presence of a cobalt-based catalyst until a conversion of DIB reaches 50 to 85% (first stage reaction step);

a step of separating unreacted DIB from a reaction product obtained in the first stage reaction step (unreacted DIB separation step); and

a step of allowing the unreacted DIB separated to react with carbon monoxide and hydrogen at 180°C or less in the presence of a cobalt-based catalyst (second stage reaction step).

[7] A method for improving a production ratio of 3,5,5-trimethylhexanal to 3,5,5-trimethylhexanol (3,5,5-trimethylhexanal/3,5,5-trimethylhexanol), in a method for producing 3,5,5-trimethylhexanal by allowing diisobutylene (DIB), carbon monoxide and hydrogen to react with one another in the presence of a cobalt-based catalyst, comprising:

a step of allowing DIB to react with carbon monoxide and hydrogen in the presence of a cobalt-based catalyst until a conversion of DIB reaches 50 to 85% (first stage reaction step);
a step of separating unreacted DIB from a reaction product obtained in the first stage reaction step (unreacted DIB separation step); and
a step of allowing the unreacted DIB separated to react with carbon monoxide and hydrogen at 180°C or less in the presence of a cobalt-based catalyst (second stage reaction step).

Advantageous Effect of Invention

By the present invention, a method for producing, selectively and in a high yield, 3,5,5-trimethylhexanal that is useful as a raw material or the like of carboxylic acid or the like that becomes a raw material of a refrigerating machine oil, etc. can be provided.

Brief Description of Drawing

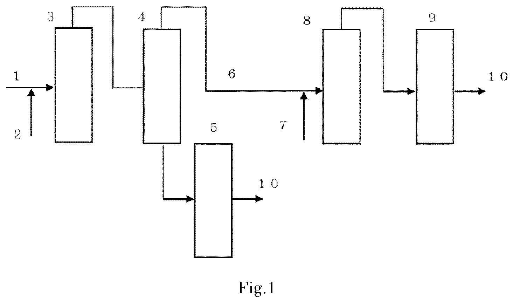

[Figure 1] Figure 1 shows an example of a flow in the case where the production method of the present invention is carried out using an industrial continuous reaction apparatus.

Description of Embodiment

Hereinafter, an embodiment for carrying out the present invention (also referred to as "present embodiment" hereinafter) will be described in detail. However, the present invention is not limited to the present embodiment, and various modifications can be made within the gist thereof.
The method for producing 3,5-5-trimethyhexanal according to the present embodiment is a method for producing 3,5,5-trimethylhexanal by allowing diisobutylene (DIB), carbon monoxide and hydrogen to react with one another in the presence of a cobalt-based catalyst, comprising

a step of allowing DIB to react with carbon monoxide and hydrogen in the presence of a cobalt-based catalyst until a conversion of DIB reaches 50 to 85% (first stage reaction step),
a step of separating unreacted DIB from a reaction product obtained in the first stage reaction step (unreacted DIB separation step), and
a step of allowing the unreacted DIB separated to react with carbon monoxide and hydrogen in the presence of a cobalt-based catalyst (second stage reaction step).

The 3,5,5-trimethylhexanal (also referred to as a "formyl form" hereinafter) obtained in the production method of the present embodiment may be one having any steric structure, or may be a mixture thereof.
The method for producing a formyl form according to the present embodiment will be described hereinafter.
In the method for producing a formyl form according to the present embodiment, the formyl form is produced by a method including three stage steps including two stage reaction steps described later using DIB as a raw material.

(First stage reaction step)

The first stage reaction step in the method for producing a formyl form according to the present embodiment is a step of allowing DIB to react with carbon monoxide and hydrogen in the presence of a cobalt-based catalyst until a conversion of DIB reaches 50 to 85%. In the present step, DIB is allowed to react with carbon monoxide and hydrogen in the presence of a cobalt-based catalyst (hydroformylation reaction), and the reaction is stopped when the conversion of DIB has reached 50 to 85%.
As the DIB that is a raw material, a commercial product may be used, or may be produced in accordance with a known method. When it is produced in accordance with a known method, it can be produced by allowing isobutene from a C4 fraction produced by fluid catalytic cracking (FCC) or in an ethylene plant to selectively react with sulfuric acid, separating isobutyl sulfate, and thereafter subjecting it to thermal decomposition.
As the DIB, 2,2,4-trimethyl-1-pentene (TM-1), 2,2,4-trimethyl-2-pentene (TM-2), or a mixture of them can be used. When a mixture of them is used, the mixing ratio is not particularly limited, but from the viewpoint of reactivity, a TM-1/TM-2 ratio is preferably 0.5 or more, more preferably 1.0 or more, and still more preferably 2.0 or more.
In the DIB as a raw material, other components, such as a C8 olefin produced as a by-product from 1-butene or 2-butene contained in the raw material isobutylene in the production of DIB may be partially contained, in addition to the aforementioned TM-1 and TM-2.

In the hydroformylation reaction, a cobalt-based catalyst used for general hydroformylation reaction is employable. Examples of the cobalt-based catalysts include an oxide, an acetylacetonate salt, various carboxylic acid salts, a sodium salt, a chloride, and a carbonyl complex, of cobalt. Specific examples thereof include carbonyl complexes of cobalt, such as $CO_2(CO)_8$, $Co_4(CO)_{12}$, $Co_6(CO)_{16}$, $NaCo(CO)_4$, $HCo(CO)_4$, and $[Co(CO)_3(C_5H_5)]_2$ (wherein $C_5H_5$ represents a cyclopentadienyl group), cobalt oxide, cobalt hydroxide, cobalt acetate, and cobalt 2-ethylhexanoate. Among these, carbonyl complexes of cobalt and cobalt hydroxide are preferable. These cobalt-based catalysts may be used singly or may be used in combination of two or more.

The cobalt concentration based on DIB is usually in the range of 0.01 to 15 g/kg, preferably 0.1 to 10 g/kg, and more preferably 0.3 to 5 g/kg. When the cobalt concentration based on DIB is 15 g/kg or less, reaction control is facilitated, and the reaction tends to be easily stopped at the desired conversion. Moreover, a precipitate of the catalyst tends to be hardly formed. On the other hand, when the cobalt concentration based on DIB is 0.01 g/kg or more, a decrease in reaction rate is suppressed, and the reaction time required for carrying out the reaction up to the desired conversion tends to be shortened.

It is possible to carry out the hydroformylation reaction without using a solvent, but a solvent is also employable. The solvent is not particularly limited as long as it dissolves DIB and the cobalt-based catalyst. Specific examples of the solvents include alcohols, such as ethanol, isopropyl alcohol, butanol, 2-ethylhexanol, and 2-octanol; esters, such as butyl acetate, cyclohexyl acetate, dibutyl phthalate, di(2-ethylhexyl) phthalate, diisononyl phthalate, diisodecyl phthalate, and triisononyl trimellitate; saturated aliphatic hydrocarbons, such as pentane, hexane, heptane, octane, isooctane, decane, dodecane, and tetradecane; alicyclic hydrocarbons, such as cyclohexane, methycyclohexane, dimethylcyclohexane, cyclooctane, cyclododecane, and decalin; aromatic hydrocarbons, such as benzene, toluene, xylene, and alkylnaphthalene; ethers, such as dibutyl ether and tetrahydrofuran; and nitriles, such as acetonitrile and propionitrile. These solvents may be used singly or may be used in combination of two or more.

The temperature of the hydroformylation reaction is usually in the range of 40 to 180°C, preferably 80 to 170°C, and more preferably 100 to 160°C. When the reaction is carried out at a temperature of 40°C or more, the reaction rate tends to be improved. On the other hand, when the reaction is carried out at a temperature of 180°C or less, the amount of an alcohol produced through a second-order reaction from an aldehyde that is a first-order reaction product, and the amount of a by-product produced from the alcohol, such as an acetal or an ether, are decreased, and the yield of the reaction tends to be improved.

It is preferable to carry out the hydroformylation reaction under pressure with a mixed gas of carbon monoxide and hydrogen (also referred to as a "synthetic gas" hereinafter). At this time, it is also possible to introduce carbon monoxide and hydrogen each independently into the reaction system or to introduce a synthetic gas, which has been prepared in advance, into the reaction system. A mole ratio (=$H_2/CO$) of the synthetic gas introduced into the reaction system is usually in the range of 0.2 to 5.0, preferably 0.5 to 2.0, and more preferably 0.7 to 1.5. In the reaction system, gasses inert to the hydroformylation reaction, such as methane, ethane, propane, nitrogen, helium, argon, and carbonic acid gas, may coexist.

The hydroformylation reaction is carried out at a pressure usually in the range of 0.2 to 40 MPaG, preferably in the range of 5 to 35 MPaG, and more preferably in the range of 10 to 30 MPaG. When the reaction is carried out at a pressure of 0.2 MPaG or more, stability of the cobalt-based catalyst tends to increase (catalyst precipitation in the reaction system is suppressed), and when the reaction is carried out at a pressure of 40 MPaG or less, initial investment for equipment tends to be able to be suppressed.

The reaction in the first stage reaction step is stopped when the conversion of DIB has reached 50 to 85%, but from the viewpoint of suppression of production of an alcohol, etc., it is preferable to stop the reaction when the conversion has reached 50 to 80%, it is more preferable to stop the reaction when the conversion has reached 50 to 75%, and it is still more preferable to stop the reaction when the conversion has reached 60 to 75%. The conversion of DIB is calculated from an analysis value of gas chromatography or the like, and specifically, it can be determined in accordance with a method described in Examples described later.

In the first stage reaction step, it is preferable to perform a neutralization and washing step after the hydroformylation reaction. In the neutralization and washing step, for example, after completion of the hydroformylation reaction, the cobalt-based catalyst can be extracted and removed by adding an aqueous solution of an alkali metal compound or an alkaline earth metal compound into the system. Examples of the alkali metal compounds and the alkaline earth metal compounds include hydroxides of lithium, sodium, potassium, magnesium and calcium, and metal salts.

In the first stage reaction step, the reaction mode of the hydroformylation reaction is not particularly limited, and the reaction can be carried out batchwise or continuously using a known reaction apparatus. As the reaction apparatus, specifically, any of a stirred reactor, a tower type reactor and a tubular reactor can be used to carry out the reaction.

(Unreacted DIB separation step)

In the method for producing a formyl form according to the present embodiment, a step of separating unreacted DIB from a reaction product obtained in the first stage reaction step (unreacted DIB separation step) is carried out. In the present step, from the reaction composition obtained in the first stage reaction step or the reaction composition having been subjected to cobalt-based catalyst removal treatment, unreacted DIB can be separated by a known purification method.

Examples of the known purification methods include distillation, absorption, and chromatography. Among these, distillation is preferable because a stationary phase requiring replacement is unnecessary, and vacuum distillation is more preferable. The conditions in the distillation operation are not particularly limited.

In the unreacted DIB after the purification, paraffin may be contained, and it may be used, as it is, as a raw material of the second stage reaction step.

(Second stage reaction step)

In the method for producing a formyl form according to the present embodiment, a step of allowing the unreacted DIB separated in the unreacted DIB separation step to react with carbon monoxide and hydrogen in the presence of a cobalt-based catalyst (second stage reaction step) is carried out. In the present step, the unreacted DIB separated is subjected to hydroformylation reaction.

Here, the DIB to be subjected to the hydroformylation reaction may be a mixture obtained by adding new DIB to the unreacted DIB separated. From the viewpoint that an aldehyde is obtained more selectively and in a higher yield, the amount of the new DIB to be mixed is preferably 75 mass% or less, more preferably 50 mass% or less, still more preferably 30 mass% or less, and particularly preferably 15 mass% or less, of the total amount of the unreacted DIB and the new DIB.

The mole ratios of the cobalt-based catalyst, the solvent and the synthetic gas during the hydroformylation reaction in the second stage reaction step can be selected according to the conditions of the first stage reaction step.

The pressure during the hydroformylation reaction is usually 0.2 to 40 MPaG, preferably 5 to 35 MPaG, more preferably 10 to 30 MPaG, and still more preferably 15 to 30 MPaG, for the same reason as in the first stage reaction step.

In the second stage reaction step, it is preferable that during the hydroformylation reaction, the cobalt concentration based on DIB be 0.1 to 20 g/kg and the reaction temperature be in the range of 100 to 210°C. Above all, it is more preferable that the reaction temperature (°C) x and the cobalt concentration (g/kg) y based on DIB satisfy the following expressions (1) and (2).

$$(1) \quad y \leq 8.0 \times 10^6 e^{-0.09x}$$

$$(2) \quad 0.1 \leq y \leq 20$$

When the reaction temperature (°C) x and the cobalt concentration (g/kg) y based on DIB satisfy the above expressions (1) and (2), production of an alcohol is suppressed, and an aldehyde tends to be obtained in a high yield.

From the viewpoints of ease of reaction control and stability of the catalyst, it is still more preferable that the cobalt concentration (g/kg) y based on DIB satisfy the following expression (3).

$$(3) \quad 0.1 \leq y \leq 15$$

From another angle, the reaction temperature during the hydroformylation reaction in the second stage reaction step is preferably 180°C or less, more preferably 170°C or less, and still more preferably 165°C or less, from the viewpoint of suppressing production of an alcohol.

In the second stage reaction step, it is preferable to stop the reaction when the conversion of the unreacted DIB has reached 60 to 100%, and it is more preferable to stop the reaction when the conversion has reached 70 to 95%, from the viewpoint of obtaining an aldehyde in a high yield.

After completion of the second stage reaction step, it is preferable to carry out removal of the cobalt-based catalyst by the same method as described in the first stage reaction step.

In the second stage reaction step, the reaction mode of the hydroformylation reaction is not particularly limited, and the reaction can be carried out batchwise or continuously using a known reaction apparatus. As the reaction apparatus, specifically, any of a stirred reactor, a tower type reactor and a tubular reactor can be used to carry out the reaction.

From the reaction composition after the first stage reaction step, the composition containing crude aldehyde from which unreacted DIB and the like have been separated, the reaction composition obtained in the second stage reaction step, or the reaction composition having been subjected to cobalt-based catalyst removal treatment, a formyl form can be purified by a known purification method. At this time, the composition derived from the first stage reaction step and the composition derived from the second stage reaction step may be separately purified, or may be purified together. Examples of the known purification methods include adsorption, extraction, neutralization and washing, distillation, adsorption, chromatography, and crystallization, and these methods may be appropriately combined. Among these, distillation is preferable, and vacuum distillation is more preferable. The conditions in the distillation operation are not particularly limited.

In Figure 1, an example of a flow in the case where the method for producing a formyl form according to the present embodiment is carried out using an industrial continuous reaction apparatus is shown.

DIB that is a raw material is fed to a first stage reactor 3 through a raw material feed pipe 1, and a mixed gas of hydrogen and carbon monoxide is fed thereto through a pipe 2. A liquid that has left the reactor 3 is separated into a liquid containing unreacted DIB and a liquid containing a formyl form in an unreacted DIB recovery apparatus 4. The liquid containing a formyl form is purified in an aldehyde purification apparatus 5, thereby obtaining purified aldehyde 10.

The liquid containing unreacted DIB is fed to a second stage reactor 8 through a second stage reaction raw material feed pipe 6, and a mixed gas of hydrogen and carbon monoxide is fed thereto through a pipe 7. A liquid that has left the reactor 8 is purified in an aldehyde purification apparatus 9, thereby obtaining purified aldehyde 10.

From the liquid that has left the first stage reactor 3, the catalyst is separated in a catalyst separation apparatus (not shown in the figure), and the liquid from which the catalyst has been separated may be fed to the unreacted DIB recovery apparatus 4. The liquid to be fed to the second stage reactor 8 may be mixed with new DIB, and thereto may be added a catalyst by a second stage reaction catalyst addition apparatus (not shown in the figure), and from the liquid that has left the reactor 8, the catalyst is separated by a catalyst separation apparatus (not shown in the figure), and the liquid from which the catalyst has been separated may be fed to the aldehyde purification apparatus 9.

According to the method for producing a formyl form of the present embodiment, 3,5,5-trimethylhexanal can be produced from DIB selectively and in a high yield.

The 3,5,5-trimethylhexanal obtained by the method for producing a formyl form of the present embodiment is not only useful as a raw material of carboxylic acid or the like that becomes a raw material of a refrigerating machine oil or the like but also employable as a raw material of an alcohol, an aldol, an ester, an amine, etc.

The present disclosure includes a method for suppressing production of 3,5,5-trimethylhexanol in a method for producing 3,5,5-trimethylhexanal by allowing DIB, carbon monoxide and hydrogen to react with one another in the presence of a cobalt-based catalyst, the method for suppressing production of 3,5,5-trimethylhexanol comprising:

a step of allowing DIB to react with carbon monoxide and hydrogen in the presence of a cobalt-based catalyst until a conversion of DIB reaches 50 to 85% (first stage reaction step);
a step of separating unreacted DIB from a reaction product obtained in the first stage reaction step (unreacted DIB separation step); and
a step of allowing the unreacted DIB separated to react with carbon monoxide and hydrogen at 180°C or less in the presence of a cobalt-based catalyst (second stage reaction step).

Moreover, the present disclosure includes a method for improving a production ratio of 3,5,5-trimethylhexanal to 3,5,5-trimethylhexanol (3,5,5-trimethylhexanal/3,5,5-trimethylhexanol) in a method for producing 3,5,5-trimethylhexanal by allowing DIB, carbon monoxide and hydrogen to react with one another in the presence of a cobalt-based catalyst, the method for improving a production ratio of 3,5,5-trimethylhexanal to 3,5,5-trimethylhexanol comprising:

a step of allowing DIB to react with carbon monoxide and hydrogen in the presence of a cobalt-based catalyst until a conversion of DIB reaches 50 to 85% (first stage reaction step);
a step of separating unreacted DIB from a reaction product obtained in the first stage reaction step (unreacted DIB separation step); and
a step of allowing the unreacted DIB separated to react with carbon monoxide and hydrogen at 180°C or less in the presence of a cobalt-based catalyst (second stage reaction step).

The production ratio of 3,5,5-trimethylhexanal to 3,5,5-trimethylhexanol (3,5,5-trimethylhexanal/3,5,5-trimethylhexanol) in the above method is preferably 30 or more, more preferably 40 or more, and still more preferably 50 or more.

The method for producing 3,5,5-trimethylhexanoic acid according to the present embodiment comprises a step of oxidizing 3,5,5-trimethylhexanal obtained in the above.

The 3,5,5-trimethylhexanoic acid obtained by the production method of the present embodiment may be one having any steric structure or may be a mixture thereof.

The step of oxidizing 3,5,5-trimethylhexanal can be carried out by a usual oxidation method, and for example, it can be carried out in accordance with a method described in Japanese Patent Laid-Open No. 2001-11009.

Examples

Hereinafter, the present invention will be more specifically described with examples, but the present invention is not limited to the following examples.

Conversion of DIB, reaction solution composition after the reaction, aldehyde yield and alcohol yield were analyzed by gas chromatography with flame ionization detector.

[Gas chromatography with flame ionization detector]

<Measurement conditions in purity analysis>

·Apparatus: Gas Chromatograph GC-2014 manufactured by SHIMADZU CORPORATION
·Column: DB-1 manufactured by Agilent Technologies, Inc. (column length: 30 m, inner diameter: 0.25 mm, film thickness: 0.25 μm)
·Temperature rise conditions: The temperature was kept at 40°C for 10 minutes, thereafter raised up to 320°C at a temperature rise rate of 8°C/min, and kept for 15 minutes.
·Temperature of sample injection portion: 320°C
·Temperature of detector: 330°C

[Example 1]

(First stage reaction step A)

A 500 ml SUS316 autoclave was charged with 213.3 g of DIB (composition: 2,2,4-trimethyl-1-pentene 76 mass%, 2,2,4-trimethyl-2-pentene 21 mass%, others 3 mass%) containing $HCo(CO)_4$ dissolved (cobalt concentration based on DIB: 1.0 g/kg), and the autoclave was purged with nitrogen. Subsequently, the autoclave was purged with a mixed gas of hydrogen and carbon monoxide ($H_2/CO=1.3$), and thereafter, the above mixed gas was fed so that the pressure might become 16 MPaG. This autoclave was placed in an electric furnace and gradually heated, and after the temperature reached 150°C, the above mixed gas was further fed so that the pressure in the autoclave might become constant at 20 MPaG, and the reaction was carried out for 0.8 hour. When the measurement was carried out by gas chromatography, a conversion of DIB after the reaction was 74%. The reaction solution composition measured by gas chromatography at this time is set forth in Table 1.

(Second stage reaction step)

As a raw material in the second stage reaction step, a made-up solution was prepared, assuming that low-boiling components (DIB, C8 paraffin) were recovered from the reaction solution of the first stage reaction step A through distillation purification, and it was used. The composition of the made-up solution A is set forth in Table 2.

A 500 ml SUS316 autoclave was charged with 213.3 g of the made-up solution A containing $HCo(CO)_4$ dissolved (cobalt concentration based on DIB: 10.8 g/kg), and the autoclave was purged with nitrogen. Subsequently, the autoclave was purged with a mixed gas of hydrogen and carbon monoxide ($H_2/CO=1.3$), and thereafter, the above mixed gas was fed so that the pressure might become 16 MPaG. This autoclave was placed in an electric furnace and gradually heated, and after the temperature reached 120°C, the above mixed gas was further fed so that the pressure in the autoclave might become constant at 20 MPaG, and the reaction was carried out for 4 hours. When the measurement was carried out by gas chromatography, a conversion of DIB after the reaction was 90.7%, an aldehyde yield was 65.1%, and an alcohol yield was 0.9%. Overall through two stages, an aldehyde yield was 75.8%, an alcohol yield was 1.0%, and an aldehyde/alcohol ratio was 73.8.

[Example 2]

The same reaction as in the second stage reaction step of Example 1 was carried out, except that the cobalt concentration based on DIB in the second stage reaction step was set to 5.3 g/kg, the reaction temperature was set to 130°C, and the reaction time was set to 3 hours.

A conversion of DIB after the reaction was 90.5%, an aldehyde yield was 65.6%, and an alcohol yield was 1.5%. Overall through two stages, an aldehyde yield was 76.0%, an alcohol yield was 1.2%, and an aldehyde/alcohol ratio was 63.8.

[Example 3]

The same reaction as in the second stage reaction step of Example 1 was carried out, except that the cobalt concentration based on DIB in the second stage reaction step was set to 11.1 g/kg, the reaction temperature was set to 140°C, and the reaction time was set to 1 hour. A conversion of DIB after the reaction was 95.5%, an aldehyde yield was 67.6%, and an alcohol yield was 2.7%. Overall through two stages, an aldehyde yield was 76.5%, an alcohol yield was 1.5%, and an aldehyde/alcohol ratio was 51.1.

[Example 4]

The same reaction as in the second stage reaction step of Example 1 was carried out, except that the cobalt concentration based on DIB in the second stage reaction step was set to 1.4 g/kg, the reaction temperature was set to 140°C, and the reaction time was set to 4 hours. A conversion of DIB after the reaction was 89.3%, an aldehyde yield was 66.3%, and an alcohol yield was 1.6%. Overall through two stages, an aldehyde yield was 76.1%, an alcohol yield was 1.2%, and an aldehyde/alcohol ratio was 62.6.

[Example 5]

The same reaction as in the second stage reaction step of Example 1 was carried out, except that the cobalt concentration based on DIB in the second stage reaction step was set to 2.8 g/kg, the reaction temperature was set to 150°C, and the reaction time was set to 1 hour. A conversion of DIB after the reaction was 89.8%, an aldehyde yield was 66.5%, and an alcohol yield was 2.3%. Overall through two stages, an aldehyde yield was 76.2%, an alcohol yield was 1.4%, and an aldehyde/alcohol ratio was 54.8.

[Example 6]

The same reaction as in the second stage reaction step of Example 1 was carried out, except that the cobalt concentration based on DIB in the second stage reaction step was set to 1.3 g/kg, the reaction temperature was set to 150°C, and the reaction time was set to 2 hours. A conversion of DIB after the reaction was 90.4%, an aldehyde yield was 63.1%, and an alcohol yield was 2.1%. Overall through two stages, an aldehyde yield was 75.3%, an alcohol yield was 1.3%, and an aldehyde/alcohol ratio was 56.2.

[Example 7]

The same reaction as in the second stage reaction step of Example 1 was carried out, except that the cobalt concentration based on DIB in the second stage reaction step was set to 0.71 g/kg, the reaction temperature was set to 150°C, and the reaction time was set to 4 hours. A conversion of DIB after the reaction was 91.4%, an aldehyde yield was 67.1%, and an alcohol yield was 2.4%. Overall through two stages, an aldehyde yield was 76.4%, an alcohol yield was 1.4%, and an aldehyde/alcohol ratio was 53.5.

[Example 8]

The same reaction as in the second stage reaction step of Example 1 was carried out, except that the cobalt concentration based on DIB in the second stage reaction step was set to 1.4 g/kg, the reaction temperature was set to 160°C, and the reaction time was set to 0.5 hour. A conversion of DIB after the reaction was 86.5%, an aldehyde yield was 56.2%, and an alcohol yield was 1.5%. Overall through two stages, an aldehyde yield was 73.5%, an alcohol yield was 1.2%, and an aldehyde/alcohol ratio was 61.0.

[Example 9]

The same reaction as in the second stage reaction step of Example 1 was carried out, except that the cobalt concentration based on DIB in the second stage reaction step was set to 0.68 g/kg, the reaction temperature was set to 160°C, and the reaction time was set to 2 hours. A conversion of DIB after the reaction was 90.8%, an aldehyde yield was 64.3%, and an alcohol yield was 2.6%. Overall through two stages, an aldehyde yield was 75.6%, an alcohol yield was 1.5%, and an aldehyde/alcohol ratio was 50.9.

[Example 10]

The same reaction as in the second stage reaction step of Example 1 was carried out, except that the cobalt concentration based on DIB in the second stage reaction step was set to 1.4 g/kg, the reaction temperature was set to 170°C, and the reaction time was set to 0.5 hour. A conversion of DIB after the reaction was 88.5%, an aldehyde yield was 64.0%, and an alcohol yield was 3.6%. Overall through two stages, an aldehyde yield was 75.6%, an alcohol yield was 1.7%, and an aldehyde/alcohol ratio was 43.6.

[Example 11]

The same reaction as in the second stage reaction step of Example 1 was carried out, except that the cobalt concentration based on DIB in the second stage reaction step was set to 0.13 g/kg, the reaction temperature was set to 170°C, and the reaction time was set to 5 hours. A conversion of DIB after the reaction was 89.5%, an aldehyde yield was 65.4%, and an alcohol yield was 3.3%. Overall through two stages, an aldehyde yield was 75.9%, an alcohol yield was 1.7%, and an aldehyde/alcohol ratio was 45.5.

[Example 12]

The same reaction as in the second stage reaction step of Example 1 was carried out, except that the cobalt concentration based on DIB in the second stage reaction step was set to 0.41 g/kg, the reaction temperature was set to 180°C, and the reaction time was set to 1 hour. A conversion of DIB after the reaction was 90.4%, an aldehyde yield was 57.3%, and an alcohol yield was 4.6%. Overall through two stages, an aldehyde yield was 73.7%, an alcohol yield was 2.0%, and an aldehyde/alcohol ratio was 36.5.

[Example 13]

The same reaction as in the second stage reaction step of Example 1 was carried out, except that the cobalt concentration based on DIB in the second stage reaction step was set to 0.12 g/kg, the reaction temperature was set to 190°C, and the reaction time was set to 2 hours. A conversion of DIB after the reaction was 88.0%, an aldehyde yield was 56.7%, and an alcohol yield was 5.8%. Overall through two stages, an aldehyde yield was 73.6%, an alcohol yield was 2.3%, and an aldehyde/alcohol ratio was 31.6.

[Example 14]

(First stage reaction step B)

A 500 ml SUS316 autoclave was charged with 213.3 g of DIB (composition: 2,2,4-trimethyl-1-pentene 76 mass%, 2,2,4-trimethyl-2-pentene 21 mass%, others 3 mass%) containing $HCo(CO)_4$ dissolved (cobalt concentration based on DIB: 1.0 g/kg), and the autoclave was purged with nitrogen. Subsequently, the autoclave was purged with a mixed gas of hydrogen and carbon monoxide ($H_2/CO=1.3$), and thereafter, the above mixed gas was fed so that the pressure might become 16 MPaG. This autoclave was placed in an electric furnace and gradually heated, and after the temperature reached 150°C, the above mixed gas was further fed so that the pressure in the autoclave might become constant at 20 MPaG, and the reaction was carried out for 0.5 hour. When the measurement was carried out by gas chromatography, a conversion of DIB after the reaction was 54%. The reaction solution composition measured by gas chromatography at this time is set forth in Table 1.

(Second stage reaction step)

As a raw material in the second stage reaction step, a made-up solution was prepared, assuming that low-boiling components (DIB, C8 paraffin) were recovered from the reaction solution of the first stage reaction step B through distillation purification, and it was used. The composition of the made-up solution B is set forth in Table 2.
The same reaction as in the second stage reaction step of Example 1 was carried out, except that the made-up solution B was used instead of the made-up solution A, the cobalt concentration based on DIB was set to 1.3 g/kg, the reaction temperature was set to 150°C, and the reaction time was set to 2 hours. A conversion of DIB after the reaction was 91.5%, an aldehyde yield was 67.0%, and an alcohol yield was 2.3%. Overall through two stages, an aldehyde yield was 72.5%, an alcohol yield was 1.3%, and an aldehyde/alcohol ratio was 54.9.

[Table 1]

|  |  | Example 1 (first stage reaction step A) | Example 14 (first stage reaction step B) |
|---|---|---|---|
| DIB | TM-1 | 14.94 | 28.91 |
|  | TM-2 | 12.98 | 17.12 |
| C8 paraffin | | 9.46 | 7.66 |
| Formyl form | | 54.09 | 41.65 |
| C9 alcohol | | 0.50 | 0.27 |
| Formic acid ester | | 0.55 | 0.24 |
| High-boiling component | | 7.48 | 4.15 |
| Total | | 100.00 | 100.00 |
| 1)Unit of numerals in the table is mass%.<br>2)TM-1:2,2,4-trimethyl-1-pentene<br>3)TM-2:2,2,4-trimethyl-2-pentene | | | |

[Table 2]

|  |  | Example 1 (made-up solution A) | Example 14 (made-up s olution B) |
|---|---|---|---|
| DIB | TM-1 | 38.73 | 52.22 |
|  | TM-2 | 32.50 | 30.96 |
| C8 paraffin | | 25.08 | 14.80 |
| Octene isomer m ixture, etc. | | 3.69 | 2.02 |
| Total | | 100.00 | 100.00 |
| 1)Unit of numerals in the table is mass%.<br>2)TM-1:2,2,4-trimethyl-1-pentene<br>3)TM-2:2,2,4-trimethyl-2-pentene | | | |

[Comparative Example 1 (one stage method)]

A 1000 ml SUS316 autoclave was charged with 430.0 g of DIB (composition: 2,2,4-trimethyl-1-pentene 76 mass%, 2,2,4-trimethyl-2-pentene 21 mass%, others 3 mass%) containing $HCo(CO)_4$ dissolved (cobalt concentration based on DIB: 2.1 g/kg), and the autoclave was purged with nitrogen. Subsequently, the autoclave was purged with a mixed gas of hydrogen and carbon monoxide ($H_2$/CO=1.1), and thereafter, the above mixed gas was fed so that the pressure might become 16 MPaG. This autoclave was placed in an electric furnace and gradually heated, and after the temperature reached 145°C, the above mixed gas was further fed so that the pressure in the autoclave might become constant at 20 MPaG. When the reaction was carried out for 2 hours, a conversion was 96.9%. At this time, an aldehyde yield was 65.7%, an alcohol yield was 2.5%, and an aldehyde/alcohol ratio was 26.8.

Overall compositions in Examples 1 to 14 and Comparative Example 1 are set forth in Table 3 and Table 4. The overall composition in each example indicates a total composition of the reaction compositions obtained in the first stage reaction step and the second stage reaction step.

[Table 3]

| | | Example 1 | Example 2 | Example 3 | Example 4 | Example 5 | Example 6 | Example 7 | Example 8 |
|---|---|---|---|---|---|---|---|---|---|
| Reaction temperature (°C) | | 120 | 130 | 140 | 140 | 150 | 150 | 150 | 160 |
| Cobalt concentration (g/kg) | | 10.8 | 5.3 | 11.1 | 1.4 | 2.8 | 1.3 | 0.71 | 1.4 |
| Second stage reaction step Conversion (%) | | 90.7 | 90.5 | 95.5 | 89.3 | 89.8 | 90.4 | 91.4 | 86.5 |
| overall composition | DIB | 2.44 | 2.49 | 1.19 | 2.80 | 2.68 | 2.53 | 2.27 | 3.54 |
| | C8 paraffin | 13.07 | 13.88 | 12.83 | 12.72 | 12.69 | 13.42 | 12.23 | 12.83 |
| | Formyl form | 75.81 | 75.97 | 76.49 | 76.14 | 76.19 | 75.32 | 76.35 | 73.49 |
| | C9 alcohol | 1.03 | 1.19 | 1.50 | 1.22 | 1.39 | 1.34 | 1.43 | 1.21 |
| | Formic acid ester | 1.30 | 1.35 | 1.54 | 1.35 | 1.30 | 1.26 | 1.44 | 1.10 |
| | High-boiling component | 6.35 | 5.12 | 6.45 | 5.77 | 5.75 | 6.14 | 6.28 | 7.83 |
| | Total | 100.00 | 100.00 | 100.00 | 100.00 | 100.00 | 100.00 | 100.00 | 100.00 |
| Aldehyde/alcohol ratio | | 73.8 | 63.8 | 51.1 | 62.6 | 54.8 | 56.2 | 53.5 | 61.0 |
| Total conversion (%) | | 97.6 | 97.5 | 98.8 | 97.2 | 97.3 | 97.5 | 97.7 | 96.5 |
| 1)Unit of numerals for overall composition in the table is mass%. | | | | | | | | | |

| | Example 9 | Example 10 | Example 11 | Example 12 | Example 13 | Example 14 | Comparative Example 1 |
|---|---|---|---|---|---|---|---|
| Reaction temperature (°C) | 160 | 170 | 170 | 180 | 190 | 150 | 145 |
| Cobalt concentration (g/kg) | 0.68 | 1.4 | 0.13 | 0.41 | 0.12 | 1.3 | 2.1 |
| Second stage reaction step Conversion (%) | 90.8 | 88.5 | 89.5 | 90.4 | 88.0 | 91.5 | – |
| Overall Composition — DIB | 2.40 | 3.03 | 2.75 | 2.53 | 3.17 | 3.92 | 3.06 |
| Overall Composition — C8 paraffin | 13.48 | 12.38 | 12.09 | 14.51 | 13.83 | 13.72 | 13.88 |
| Overall Composition — Formyl form | 75.61 | 75.55 | 75.92 | 73.69 | 73.62 | 72.51 | 65.74 |
| Overall Composition — C9 alcohol | 1.49 | 1.73 | 1.67 | 2.02 | 2.33 | 1.32 | 2.46 |
| Overall Composition — Formic acid ester | 1.16 | 1.23 | 1.48 | 1.39 | 1.41 | 1.26 | 2.87 |
| Overall Composition — High-boiling component | 5.86 | 6.08 | 6.09 | 5.76 | 5.64 | 7.27 | 12.00 |
| Overall Composition — Total | 100.00 | 100.00 | 100.00 | 100.00 | 100.00 | 100.00 | 100.00 |
| Aldehyde/alcohol ratio | 50.9 | 43.6 | 45.5 | 36.5 | 31.6 | 54.9 | 26.8 |
| Total conversion (%) | 97.6 | 97.0 | 97.3 | 97.5 | 96.8 | 96.1 | 96.9 |

[0072] [Table 4]

1)Unit of numerals for overall composition in the table is mass%.

From the above results, it can be seen that the production method of the present invention is a method in which an aldehyde yield is high, and an aldehyde/alcohol ratio is also high, that is to say, an alcohol that is an impurity is not easily produced, aldehyde selectivity is high, and 3,5,5-trimethylhexanal can be produced selectively and in a high yield.

[Example 15]

A 2 L glass flask was charged with 840.1 g of an aldehyde obtained by subjecting an aldehyde, which had been produced in the same manner as in Example 1, to distillation purification, and the system was purged with oxygen. Subsequently, oxygen was fed at 280 mL/min from a sparger. The glass flask was gradually heated, and after the temperature reached 60°C, the reaction was carried out for 7 hours. When the measurement was carried out by gas chromatography, a yield of 3,5,5-trimethylhexanoic acid was 92%.

Industrial Applicability

By the present invention, a method for producing, selectively and in a high yield, 3,5,5-trimethylhexanal that is useful as a raw material or the like of carboxylic acid or the like that becomes a raw material of a refrigerating machine oil, etc. can be provided.

Reference Signs List

1: Raw material feed pipe
2: Feed pipe for mixed gas of hydrogen and carbon monoxide
3: First stage reactor
4: Unreacted DIB recovery apparatus
5: Aldehyde purification apparatus
6: Second stage reaction raw material feed pipe
7: Feed pipe for mixed gas of hydrogen and carbon monoxide
8: Second stage reactor
9: Aldehyde purification apparatus
10: Purified aldehyde

Claims

1.    A method for producing 3,5,5-trimethylhexanal by allowing diisobutylene (DIB), carbon monoxide and hydrogen to react with one another in the presence of a cobalt-based catalyst, comprising:

> a step of allowing DIB to react with carbon monoxide and hydrogen in the presence of a cobalt-based catalyst until a conversion of DIB reaches 50 to 85% (first stage reaction step);
> a step of separating unreacted DIB from a reaction product obtained in the first stage reaction step (unreacted DIB separation step); and
> a step of allowing the unreacted DIB separated to react with carbon monoxide and hydrogen in the presence of a cobalt-based catalyst (second stage reaction step).

2. The method for producing 3,5,5-trimethylhexanal according to claim 1, wherein in the second stage reaction step, a reaction temperature (°C) x and a cobalt concentration (g/kg) y based on DIB satisfy the following expressions (1) and (2):

$$(1) \quad y \leq 8.0 \times 10^6 e^{-0.09x}$$

$$(2) \quad 0.1 \leq y \leq 20.$$

3. The method for producing 3,5,5-trimethylhexanal according to claim 2, wherein in the second stage reaction step, the cobalt concentration (g/kg) y based on DIB satisfies the following expression (3):

$$(3) \quad 0.1 \leq y \leq 15.$$

4. The method for producing 3,5,5-trimethylhexanal according to any one of claims 1 to 3, wherein the reaction temperature in the second stage reaction step is 180°C or less.

5. A method for producing 3,5,5-trimethylhexanoic acid, comprising a step of oxidizing 3,5,5-trimethylhexanal obtained in the production method according to any one of claims 1 to 4.

**Patentansprüche**

1. Verfahren zur Herstellung von 3,5,5-Trimethylhexanal, indem Diisobutylen (DIB), Kohlenmonoxid und Wasserstoff erlaubt wird, in Gegenwart eines kobaltbasierten Katalysators miteinander zu reagieren, umfassend:

> einen Schritt, in dem DIB erlaubt wird, in Gegenwart eines kobaltbasierten Katalysators mit Kohlenmonoxid und Wasserstoff zu reagieren, bis eine Umwandlung von DIB 50 bis 85 % erreicht (Reaktionsschritt der ersten Stufe);
> einen Schritt des Abscheidens von nicht reagiertem DIB von einem Reaktionsprodukt, erhalten in dem Reaktionsschritt der ersten Stufe (Schritt des Abscheidens von nicht reagiertem DIB); und
> einen Schritt, in welchem dem abgeschiedenen nicht reagierten DIB erlaubt wird, in Gegenwart eines kobaltbasierten Katalysators mit Kohlenmonoxid und Wasserstoff zu reagieren (Reaktionsschritt der zweiten Stufe).

2. Verfahren zur Herstellung von 3,5,5-Trimethylhexanal nach Anspruch 1, wobei im Reaktionsschritt der zweiten Stufe eine Reaktionstemperatur (°C) x und eine Kobaltkonzentration (g/kg) y auf Grundlage von DIB die folgenden Ausdrücke (1) und (2) erfüllen:

$$(1) \quad y \leq 8,0 \times 10^6 e^{-0,09x}$$

$$(2) \quad 0,1 \leq y \leq 20.$$

3. Verfahren zur Herstellung von 3,5,5-Trimethylhexanal nach Anspruch 2, wobei im Reaktionsschritt der zweiten Stufe die Kobaltkonzentration (g/kg) y auf Grundlage von DIB den folgenden Ausdruck (3) erfüllt:

$$(3) \quad 0,1 \leq y \leq 15.$$

**4.** Verfahren zur Herstellung von 3,5,5-Trimethylhexanal nach einem der Ansprüche 1 bis 3, wobei die Reaktionstemperatur im Reaktionsschritt der zweiten Stufe 180 °C oder weniger ist.

**5.** Verfahren zur Herstellung von 3,5,5-Trimethylhexansäure, umfassend einen Schritt des Oxidierens von 3,5,5-Trimethylhexanal, erhalten im Herstellungsverfahren nach einem der Ansprüche 1 bis 4.

**Revendications**

**1.** Procédé de production de 3,5,5-triméthylhexanal par le fait de faire réagir du diisobutylène (DIB), du monoxyde de carbone et de l'hydrogène en présence d'un catalyseur à base de cobalt, comprenant :

une étape consistant à laisser le DIB réagir avec du monoxyde de carbone et de l'hydrogène en présence d'un catalyseur à base de cobalt jusqu'à ce que la conversion du DIB atteigne 50 à 85 % (première étape de réaction) ;
une étape de séparation du DIB qui n'a pas réagi d'un produit de réaction obtenu lors de la première étape de réaction (étape de séparation du DIB qui n'a pas réagi) ; et
une étape consistant à laisser le DIB qui n'a pas réagi et qui a été séparé réagir avec du monoxyde de carbone et de l'hydrogène en présence d'un catalyseur à base de cobalt (deuxième étape de la réaction).

**2.** Procédé de production de 3,5,5-triméthylhexanal selon la revendication 1, dans lequel, lors de la deuxième étape de réaction, une température de réaction (°C) x et une concentration en cobalt (g/kg) y par rapport au DIB satisfont les équations (1) et (2) suivantes :

$$(1)\ y \leq 8,0 \times 10^{6} e^{-0,09x}$$

$$(2)\ 0,1 \leq y \leq 20.$$

**3.** Procédé de production de 3,5,5-triméthylhexanal selon la revendication 2, dans lequel, lors de la deuxième étape de réaction, la concentration en cobalt (g/kg) y par rapport au DIB satisfait à l'équation suivante (3) :

$$(3)\ 0,1 \leq y \leq 15.$$

**4.** Procédé de production de 3,5,5-triméthylhexanal selon l'une quelconque des revendications 1 à 3, dans lequel la température de réaction lors de la deuxième étape de réaction est inférieure ou égale à 180°C.

**5.** Procédé de production de l'acide 3,5,5-triméthylhexanoïque, comprenant une étape d'oxydation du 3,5,5-triméthylhexanal obtenu dans le procédé de production selon l'une quelconque des revendications 1 à 4.

Fig.1

**EP 4 219 438 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4368454 B
- JP 2002053501 A
- GB 702204 A
- JP 2001011009 A